# EUROPÄISCHE PATENTSCHRIFT

(11) **EP 2 414 345 B1**
(45) Veröffentlichungstag und Bekanntmachung des Hinweises auf die Patenterteilung: **17.09.2014**
(21) Anmeldenummer: 10712340.8
(22) Anmeldetag: 26.03.2010
(51) Int. Cl.: C07D 401/04, A01N 43/56

(54) **VERFAHREN ZUM HERSTELLEN VON PYRIDYL-SUBSTITUIERTEN PYRAZOLEN**
Method for manufacturing Pyridyl-substituted pyrazoles
Procédé de fabrication de pyrazoles substitués par pyridile

(30) Priorität: 03.04.2009 EP 09157317
(43) Veröffentlichungstag der Anmeldung: 08.02.2012
(73) Patentinhaber: Bayer CropScience AG, 40789 Monheim (DE)
(72) Erfinder: PAZENOK, Sergii, 42699 Solingen (DE); LUI, Norbert, 51519 Odenthal (DE); BLASCHKE, Harry, deceased (DE)
(86) Internationale Anmeldenummer: PCT/EP2010/001926
(87) Internationale Veröffentlichungsnummer: WO 2010/112178

(56) Entgegenhaltungen:
- US-A1- 2006 183 785

## Beschreibung

Die vorliegende Erfindung betrifft ein Verfahren zum Herstellen von 1-(Pyridyl)-substituierten Pyrazolen umfassend die Umsetzung von Acetylenketonen mit Hydrazin-Derivaten zu 1-Pyridyl-substituierten dihydro-1H-Pyrazolen, deren Weiterreaktion unter Wasserabspaltung zu 1-Pyridyl-substituierten Trihalogenmethylpyrazolen und deren Weiterverarbeitung.

1-Pyridyl-substituierte Pyrazole und dihydro-1H-Pyrazole sind wertvolle Zwischenprodukte zur Herstellung von Anthranilsäureamiden, die als Insektizide Verwendung finden können.

In der Literatur ist bereits beschrieben, dass Pyrazole durch Reaktion von 1,3-Dicarbonylen oder entsprechenden 1,3-bis-elektrophilen Reagenzien mit Monoalkyl- oder Arylhydrazinen gebildet werden können (Synthesis 2004, N1. pp 43-52). Jedoch wird berichtet, dass im Fall von Monoalkyl- oder Monoarylhydrazinen eine Mischung aus regioisomeren Pyrazolen resultiert (Tetrahedron 59 (2003), 2197-2205; Martins et al., T. L. 45 (2004) 4935). Versuche, exklusiv ein Regioisomer zu erhalten, schlugen fehl (JOC 2007, 72822 8243-8250). In der Literatur ebenfalls beschrieben ist ein Verfahren zur Herstellung von Trifluormethyl-Pyrazolen (WO 2003/016282). Ebenfalls sind Herstellverfahren von (Het)Aryl-substituierten Pyrazolen beschrieben (WO 2007/144100), wobei durch Reduktion von Diestern mit DIBAL oder LiAIH₄ die entsprechenden Pyrazole erhalten werden. Allerdings sind dabei sehr tiefe Temperaturen erforderlich, und die Verwendung von DIBAL ist unwirtschaftlich.

Die Aufgabe der vorliegenden Erfindung ist daher die Bereitstellung neuer, wirtschaftlicher Verfahren zum Herstellen von 1-Pyridyl-substituierten Pyrazol-Derivaten und 1-Pyridyl-substituierten dihydro-1H-Pyrazolen, die die zuvor beschriebenen Nachteile nicht aufweisen.

Die Aufgabe wurde gemäß der vorliegenden Erfindung gelöst durch ein Verfahren zum Herstellen von Pyridyl-substituierten Pyrazol-Derivaten der allgemeinen Formel (I) 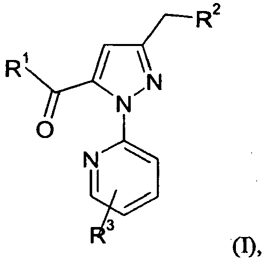 in welcher
- R¹: für Alkoxy, Hydroxy, Aryloxy, Alkylaryloxy, Alkyl, Cycloalkyl, Halogen steht,
- R²: für Hydroxy, Alkoxy, Arylalkoxy, Alkylthio, für Chlor, Brom, Fluor, Jod, O-(C=O)Alkyl, O-(C=O)O-Alkyl, OSO₂Alkyl, OSO₂Ph, OSO₂ Halogenalkyl, OSO₂-Aryl steht,
dadurch gekennzeichnet, dass man
Acetylenketone der Formel (II) 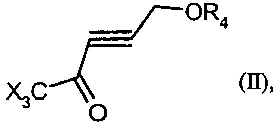 in welcher
R⁴ für eine Schutzgruppe steht, ausgewählt aus (C₁-C₆)-Alkyl, Aryl, Benzyl, Tetrahydropyran, (C=O)-Alkyl, (C=O)-O-Alkyl, Si(Alkyl)₃
und X für Halogen steht,
mit Hydrazinopyridinen der Formel (III) 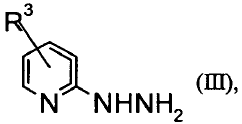 in welcher
R³ für Halogen, CN, NO₂, Alkyl, Cycloalkyl, Halogenalkyl, Halogencycloalkyl, Alkoxy, Halogenalkoxy, Alkylamino, Dialkylamino, Cycloalkylamino, steht,
umsetzt zu 1-Pyridyl-substituierten dihydro-1H Pyrazolen der Formel (IV), 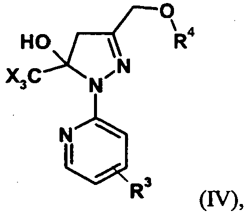 in welcher X, R³, R⁴ die oben angegebenen Bedeutungen haben,
diese gegebenenfalls ohne vorherige Isolierung unter Wasserabspaltung weiter umsetzt zu 1-Pyridyl-substituierten -Trihalogenmethylpyrazolen der Formel (V) 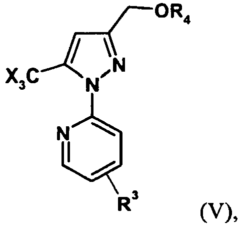 in welcher X, R³, R⁴ die oben angegebenen Bedeutungen haben,
diese Verbindungen der allgemeinen Formel (V)
unter Zugabe von beispielsweise H₂SO₄ umsetzt zu Pyrazolcarbonsäuren der Formel (VI), 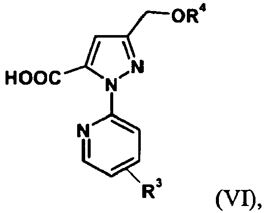 in welcher R³, R⁴ die oben angegebenen Bedeutungen haben,
diese nach Abspaltung der Schutzgruppe R⁴ zu Hydroxymethylpyrazolsäuren der Formel (VII) umsetzt, 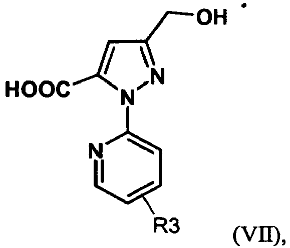 in welcher R³ die oben angegebenen Bedeutungen hat,
und diese zu Verbindungen der Formel (I) umsetzt

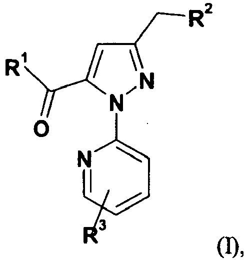

Überraschenderweise wird eine regioselektive Umsetzung von Acetylenketonen der Formel (II) mit Hydrazinopyridinen der Formel (III) beobachtet, so dass die im Stand der Technik berichteten Nachteile nicht beobachtet werden. Beispielsweise liefert die Umsetzung von 5-(Alkoxy oder Benzyloxy)1,1,1-trichloropent-3-yn-2-one mit Hydrazinpyridin in hoher Ausbeute nur das gewünschte 3-[Benzyloxymethyl]-1-(3-chloropyridin-2-yl)-5-(trichloromethyl)-4,5-dihydro-1H-pyrazol-5-ol bzw. 3-[Methyloxymethyl]-1-(3-chloropyridin-2-yl)-5-(trichloromethyl)-4,5-dihydro-1H-pyrazol-5-ol.

Das erfindungsgemäße Verfahren kann anhand des folgenden Schemas (I) erläutert werden :

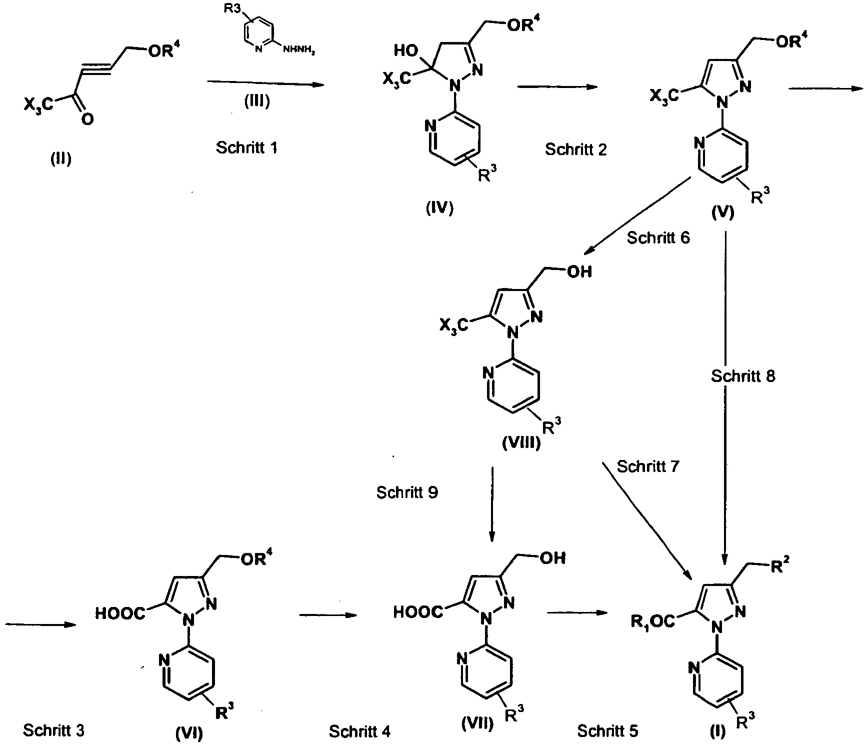

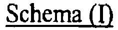

Die Umsetzung einer Verbindung der Formel (VII) zu einer Verbindung der Formel (I) wird beispielhaft anhand des nachfolgenden Schemas II erläutert:

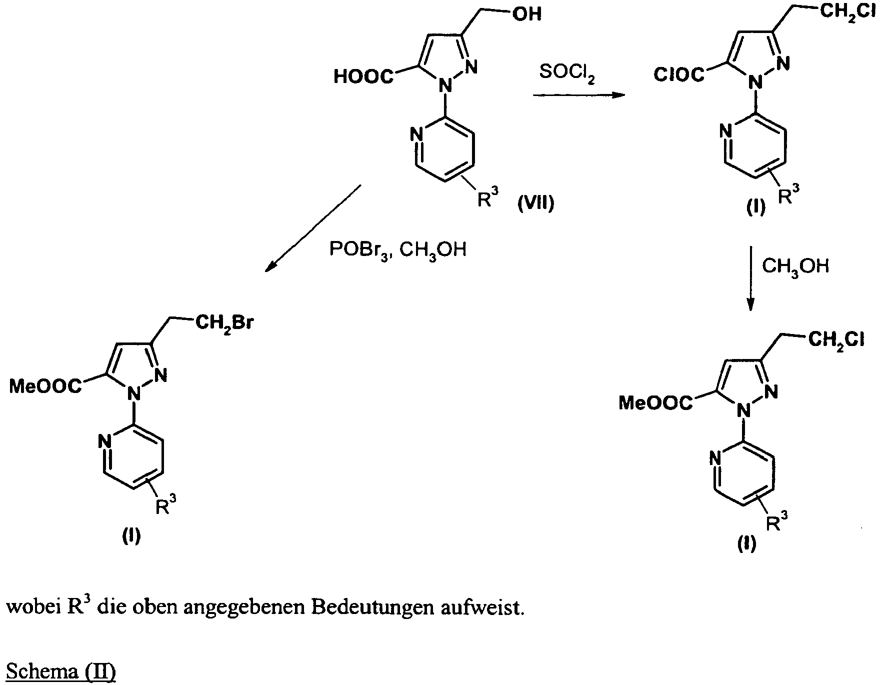

### Allgemeine Definitionen

Im Zusammenhang mit der vorliegenden Erfindung umfasst der Begriff Halogene (X), soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden. Substituierte Gruppen können einfach oder mehrfach substituiert sein, wobei bei Mehrfachsubstitutionen die Substituenten gleich oder verschieden sein können.

Mit einem oder mehreren Halogenatomen (-X) substituierte Alkyl-Gruppen (= Halogenalkyl-Gruppen) sind beispielsweise ausgewählt aus Trifluormethyl (CF₃), Difluormethyl (CHF₂), CCl₃, CFCl₂, CF₃CH₂, ClCH₂, CF₃CCl₂.

Alkyl-Gruppen sind im Zusammenhang mit der vorliegenden Erfindung, soweit nicht abweichend definiert, lineare oder verzweigte Kohlenwasserstoff-Gruppen.

Die Definition Alkyl und C₁-C₁₂-Alkyl umfasst beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Cycloalkyl-Gruppen sind im Zusammenhang mit der vorliegenden Erfindung, soweit nicht abweichend definiert, ringförmige gesättigte Kohlenwasserstoff-Gruppen.

Arylalkyl-Gruppen und Arylalkoxy-Gruppen sind im Zusammenhang mit der vorliegenden Erfindung, soweit nicht abweichend definiert, durch Aryl-Gruppen substituierte Alkyl- bzw. AlkoxyGruppen, die eine Alkylenkette aufweisen können. Im Einzelnen umfasst die Definition Arylalkyl beispielsweise die Bedeutungen Benzyl und Phenylethyl; die Definition Arylalkoxy bespielsweise die Bedeutung Benzyloxy.

Alkylaryl-Gruppen (Alkaryl-Gruppen) und Alkylaryloxy-Gruppen sind im Zusammenhang mit der vorliegenden Erfindung, soweit nicht abweichend definiert, durch Alkyl-Gruppen substituierte Aryl-Gruppen, bzw Aryloxy-Gruppen, die eine C₁₋₈-Alkylenkette aufweisen können und im Arylgerüst oder Aryloxygerüst ein oder mehrere Heteroatome, die ausgewählt sind aus O, N, P und S, aufweisen können.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart und beansprucht.

### Propargylether der Formel (II)

Die bei der Durchführung des erfindungsgemäßen Verfahrens als Ausgangsstoffe verwendeten Propargylether sind durch die Formel (II) allgemein definiert 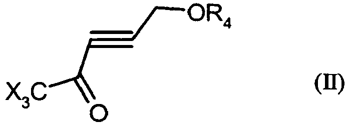 wobei X für Halogen, bevorzugt für Fluor, Chlor oder Br, ganz besonders bevorzugt für Chlor steht,
R⁴ für eine Schutzgruppe steht, ausgewählt aus (C₁-C₆)-Alkyl, Aryl, Benzyl, Tetrahydropyran, (C=O)-Alkyl, (C=O)-OAlkyl, Si(Alkyl)₃. bevorzugt für Benzyl, Si(Me)₃, Phenyl, (C₁-C₄)Alkyl, (C=O)O-tert-butyl steht, besonders bevorzugt für (C₁-C₄)-Alkyl und Benzyl und (C=O)O-tert-butyl steht.

Beispiele für erfindungsgemäß geeignete Acetylenketone der Formel (II) sind
5-(Benzyloxy)-1,1,1-trichloropent-3-yn-2-one, 5-(Benzyloxy)-1-bromo-1,1-dichloropent-3-yn-2-one, 5-(Benzyloxy)-1,1-dichloro-1-fluoropent-3-yn-2-one, 5-(Phenyloxy)-1,1,1-trichloropent-3-yn-2-one, 5-(benzyloxy)-1,1,1-trifluorpent-3-yn-2-one, 5-(benzyloxy)-1,1,1-trichloropent-3-yn-2-one, 1,1,1-trichloro-5-(tetrahydro-2H-pyran-2-yloxy)pent-3-yn-2-one, 5-(Trimethylsilyloxy)-1,1,1-trichloropent-3-yn-2-one, 5-(Methyloxy)-1,1,1-trichloropent-3-yn-2-one.

Verfahren zur Herstellung von Acetylenketone sind im Stand der Technik beschrieben, beispielsweise in THL 45(2004), 4935-4938; JOC 2002, 67, 9200-9209.

### Hydrazinopyridine der allgemeinen Formel (III)

Die gemäß der vorliegenden Erfindung verwendeten Hydrazinopyridine sind Verbindungen der allgemeinen Formel (III) 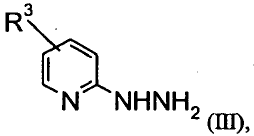 in welcher
R³ für Halogen, CN, NO₂, Alkyl, Cycloalkyl, Halogenalkyl, Halogencycloalkyl, Alkoxy, Halogenalkoxy, Alkylamino, Dialkylamino, Cycloalkylamino, steht,
R³ bevorzugt für Halogen, CN, NO₂, (C₁-C₆)-Alkyl, Halogen(C₁-C₆)-alkyl, (C₁-C₆)Alkoxy, Halogen(C₁-C₆)alkoxy, steht,
R³ besonders bevorzugt für F, Chlor, Brom, Jod, CN, (C₁-C₄)-Alkyl, Halogen(c₁-C₄)-alkyl, Halogen(C₁-C₄)alkoxy, steht,
R³ ganz besonders bevorzugt für Fluor, Chlor, Brom, Jod, insbesondere für Chlor steht.

Ein Beispiel für ein erfindungsgemäß geeignetes Hydrazinopyridine ist 3-Chloro-2-hydrazinopyridin.

### Schritt (1)

In einer ersten Ausführungsform des vorliegenden Verfahrens, werden zunächst 2-acylierte Propargylether der Formel (II) mit Hydrazinopyridinen der Formel (III) umgesetzt. Im Anschluss daran werden die im Schritt (1) gebildeten Zwischenprodukte zu den 5-Trihalogenmethylpyrazol-Derivaten der Formel (V) unter Wasser Abspaltung umgesetzt (Schritt 2). 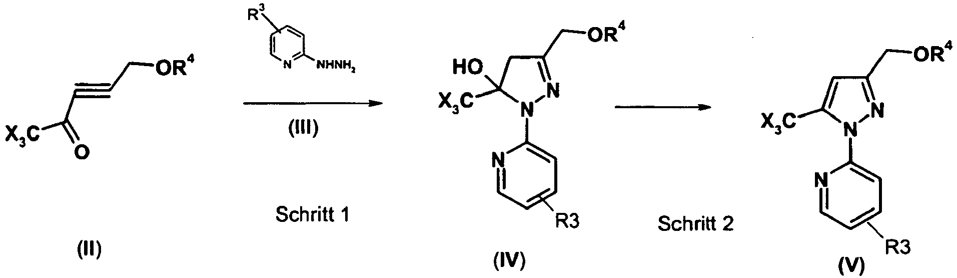 in welcher X, R³, R⁴ die oben angegebenen Bedeutungen haben.

Es ist als überraschend anzusehen, das die cyclisierung von Propargylether der Formel (II) mit Hydrazinopyridinen der Formel (III) hoch regioselektiv verläuft, sodass nur das gewünschte Regioisomer der Formel (IV) gebildet wird. Die Verbindungen der Formel (IV) und (V) sind neu.

Die Durchführung des erfindungsgemäßen Verfahrensschritts (1) erfolgt vorzugsweise innerhalb eines Temperaturbereichs von -20°C bis +100°C, besonders bevorzugt bei Temperaturen von -10°C bis +70 °C.

Der erfindungsgemäße Verfahrensschritt (1) wird im Allgemeinen unter Normaldruck durchgeführt. Alternativ ist es jedoch auch möglich, im Vakuum zu arbeiten, um das Wasser zu entfernen.

Die Reaktionszeit ist nicht kritisch und kann, in Abhängigkeit von der Ansatzgröße und Temperatur, in einem Bereich zwischen wenigen Minuten und mehreren Stunden gewählt werden.

Bei der Durchführung des erfindungsgemäßen Verfahrensschritts setzt man 1 Mol des Propargylethers der Formel (II) mit 0.8 Mol bis 1.5 Mol, vorzugsweise 0.9 Mol bis 1.2 Mol, besonders bevorzugt mit der äquimolaren Menge des Hydrazinopyridins der Formel (III) um.

Geeignete Lösungsmittel sind beispielsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan, Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfone, wie Sulfolan,Alkohole wie Methanol, Ethanol, i-Propanol. Besonders bevorzugt verwendet man Toluol, Xylol, Chlorbenzol, n-Hexan, Cyclohexan oder Methylcyclohexan, Ethanol, ganz besonders bevorzugt Toluol, Xylol, THF, Methyl-tert-butylether, Ethanol, Acetonitril.

Die gebildeten 3-[(Alkoxy)methyl]-1-(pyridin-2-yl)-5-(trihaloalkyl)-4,5-dihydro-1H-pyrazol-5-ols können ohne vorherige Aufarbeitung im darauffolgenden Schritt (2), in welchem Wasserabspaltung stattfindet, eingesetzt werden.

Alternativ können diese Zwischenprodukte durch geeignete Aufarbeitungsschritte und ggf. weitere Aufreinigung isoliert werden. Erst zu einem späteren Zeitpunkt kann dann Wasser abgespalten werden.

### Schritt 2. Wasserabspaltung

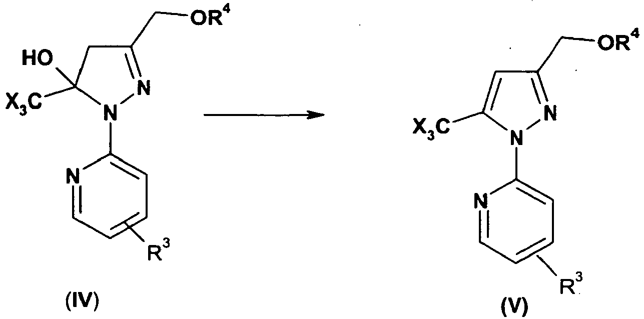

in welcher X, R³, R⁴ die oben angegebenen Bedeutungen haben.

Für die Wasserabspaltung können z.B folgende Reagenzien eingesetzt werden: H₂SO₄, CF₃COOH, (CH₃)₃COCl, POCl₃, Polyphosphorsäure, SOCl₂, (CH₃CO)₂O, (CF₃CO)₂O, Oxalylchlorid, Phosgen, Diphosgen.

Besonders bevorzugt sind (CF₃CO)₂O, Oxalylchlorid, Thionylchlorid und Phosgen.

Die Durchführung des erfindungsgemäßen Verfahrensschritts (2) erfolgt vorzugsweise innerhalb eines Temperaturbereichs von -20°C bis +100°C, besonders bevorzugt bei Temperaturen von -10°C bis +70 °C.

Der erfindungsgemäße Verfahrensschritt (2) wird im Allgemeinen unter Normaldruck durchgeführt. Alternativ ist es jedoch auch möglich, im Vakuum oder unter Überdrück (z.B. Umsetzung mit Phosgen) zu arbeiten.

Die Reaktionszeit ist nicht kritisch und kann, in Abhängigkeit von der Ansatzgröße und Temperatur, in einem Bereich zwischen wenigen Minuten und mehreren Stunden gewählt werden.

Bei der Durchführung des erfindungsgemäßen Verfahrensschritts setzt man 1 Mol des 3-[(Alkoxy)methyl]-1-(pyridin-2-yl)-5-(trihaloalkyl)-4,5-dihydro-1H-pyrazol-5-ol der Formel (IV) mit 0.1 Mol bis 2 Mol, vorzugsweise 0.2 Mol bis 1.8 Mol, besonders bevorzugt mit 0.2-1 Mol des Entwässerungsmittels um. Es ist auch möglich die Wasserabspaltung mit katalytischen Mengen von H2SO4 oder CF3COOH durchführen.

Geeignete Lösungsmittel sind beispielsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan, Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Ketone, wie Aceton, Methylethylketon, Methylisobutylketon oder Cyclohexanon; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfone, wie Sulfolan. Besonders bevorzugt verwendet man Toluol, Xylol, Chlorbenzol, n-Hexan, Cyclohexan oder Methylcyclohexan, ganz besonders bevorzugt Toluol, Xylol, THF, CH₂Cl₂, Methyl-tert-butylether.

### Schritte 3 und 4

In einer weiteren Ausführungsform des erfindungsgemäßen Verfahrens werden die Trihalogenalkylpyrazole der Formel (V) gemäß dem nachfolgenden Schema zu den Pyrazolen der Formel (VI) oder Formel (VII) umgesetzt. Dabei wird die Alkoxycarbonsäure der Formel (VI) durch Hydrolyse der Trihalogenmethylgruppe hergestellt (Schritt 3), dann wird durch Abspaltung der Schutz-Gruppe das gewünschte 3-(Hydroxymethyl)-1-(pyridin-2-yl)-1H-pyrazole-5-carbonsaüre hergestellt (Schritt 4). 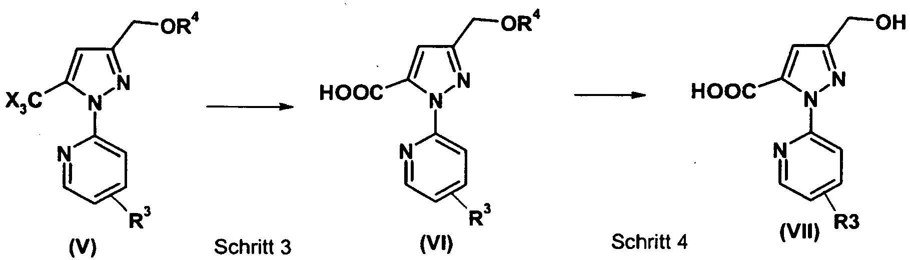 in welcher X, R³, R⁴ die oben angegebenen Bedeutungen haben.

In einer bevorzugten Ausführungsform des erfindungsgemäßen Verfahrens wird das 2-[3-(Alkoxymethyl)-5-(trihalomethyl)-1H-pyrazol-1-yl]pyridine der Formel (V) direkt zur 3-(Hydroxymethyl)-1-(pyridin-2-yl)-1H-pyrazole-5-carbonsäure der Formel (VII) umgewandelt. 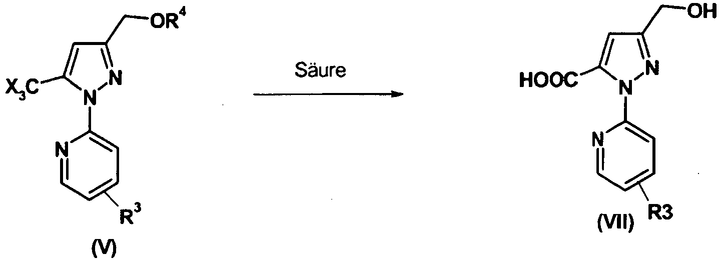 in welcher X, R³, R⁴ die oben angegebenen Bedeutungen haben.

Die Verbindungen der Formel (VII) sind ebenfalls neu.

Die Reaktion wird in der Regel unter sauren oder basischen Bedingungen durchgeführt.

Bevorzugt sind mineralische Säuren, beispielsweise H₂SO₄, HCl, HSO₃Cl, HF, HBr, HI, H₃PO₄ oder organische Säuren, beispielsweise CF₃COOH, p-Toluolsulfonsäure, Methansulfonsäure, Trifluormethansulfonsäure.

Die Reaktion kann durch der Zusatz von Katalysatoren wie beispielsweise FeCl₃, AlCl₃, BF₃, SbCl₃, NaH₂PO₄ beschleunigt werden.

Basische Hydrolyse erfolgt in Gegenwart von organischen Basen wie Trialkylamine, Alkylpyridine, Phosphazene und 1,8-Diazabicyclo[5.4.0]undecen (DBU), anorganischen Basen wie, Alkalimetallhydroxide wie z.B. Lithium-, Natrium- oder Kaliumhydroxid, Alkalimetallcarbonate (Na₂CO₃, K₂CO₃) und Acetate wie NaOAc, KOAc, LiOAc, -Alkoholate, wie z.B. NaOMe, NaOEt, NaOt-Bu, KOt-Bu.

### Schritte 6 und 9

In einer weiteren Ausführungsform des erfindungsgemäßen Verfahrens wird zuerst die Alkoxygruppe abgespalten (Schritt 6). Anschließend wird die Hydrolyse der Trihalogenmethylgruppe vorgenommen (Schritt 9). 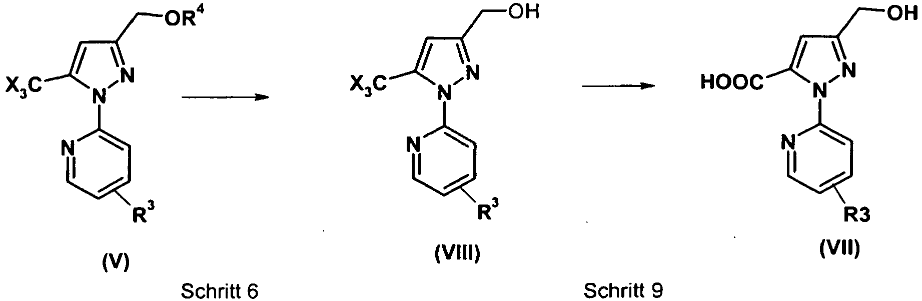 wobei X und R³ und R⁴ die oben angegebenen Bedeutungen haben.

Die Abspaltung der Schutzgruppe ist abhängig von der Definition des Restes R⁴. Falls R⁴ für (C₁-C₆)-Alkyl oder Benzyl steht, kann die Abspaltung in Gegenwart von BBr₃, HCl, HJ, Me₃SiI, PyHCl, FeCl₃, BF₃, im Fall von Benzyl zusätzlich durch katalytische Hydrierung erfolgen. Acetyl, oder Alkylsulfonyl-Gruppen können unter basischen Bedingungen (NaOH, KOH, Na₂CO₃, NaHC03) und SiMe₃ in Gegenwart von F-Anionen abgespaltet werden.

**Schritt 8.** Falls R⁴ für (C₁-C₆)Alkyl oder Benzyl steht, kann die CX₃-Gruppe direkt zur Estergruppe umgewandelt werden. Somit können Verbindungen der Formel (V) direkt in die Verbindungen der Formel (I) umgewandelt werden (Schritt 8). 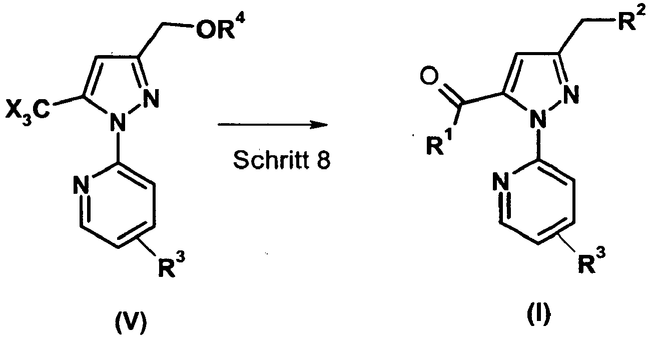 wobei
- X, R² ,R³ und R⁴: die oben angegebenen Bedeutungen aufweisen,
- R¹: für (C₁-C₆)-Alkoxy, steht,
- R¹: bevorzugt für Methoxy, Ethoxy, Propoxy steht,
- R²: für (C₁-C₆)-Alkoxy, Aryl(C₁-C₆)-alkoxy steht,
- R²: bevorzugt für (C₁-C₆)-Alkoxy steht.

Für diese Zwecke benutzt man z.B. Alkohole beispielsweise Methanol, Ethanol, Propanol oder die Kombinationen Alkohol/HCl, Alkohol/FeCl₃, Alkohol/H₂SO₄ oder Alkohol/Alkoholat (NaOMe, NaOEt, KOEt, NaOPr).

Der Reaktionsschritt 8 kann in Substanz oder in einem Lösungsmittel durchgeführt werden. Vorzugsweise wird die Reaktion in einem Lösungsmittel durchgeführt. Geeignete Lösungsmittel sind beispielsweise ausgewählt aus der Gruppe bestehend aus Wasser, aliphatischen und aromatischen Kohlenwasserstoffen, wie z.B. n-Hexan, Benzol oder Toluol, die durch Fluor- und Chloratome substituiert sein können, wie Methylenchlorid, Dichlorethan, Fluorbenzol, Chlorbenzol oder Dichlorbenzol; Ethern, wie z.B. Diethylether, Diphenylether, Methyl-tert-butylether, Isopropylethylether, Dioxan, Diglym, Dimethylglycol, Dimethoxyethane (DME) oder THF; Nitrilen wie Methylnitril, Butylnitril oder Phenylnitril; Alkohole wie Methanol, Ethanol, i-Propanol , Amide wie Dimethylformamid (DMF) oder N-methylpyrrolidon (NMP) oder Mischungen solcher Lösungsmittel geeignet, wobei das Wasser, Alkohole wie Methanol, Ethanol, i-Propanol, Acetonitril, Dichlormethan besonders gut geeignet sind.

### Schritt 7

Bei Verbindungen der Formel (VIII) kann die CX₃-Gruppe direkt zur Estergruppe umgewandelt werden. Somit können die Verbindungen der Formel (VIII) direkt in die Verbindungen der Formel (I) umgewandelt werden (Schritt 7).

### Schritt 5

Die bei der Durchführung des erfindungsgemäßen Verfahrens verwendeten Verbindungen der Formel (VII) werden in einem zweistufigen Prozess in die Verbindungen der Formel (I) umgewandelt.

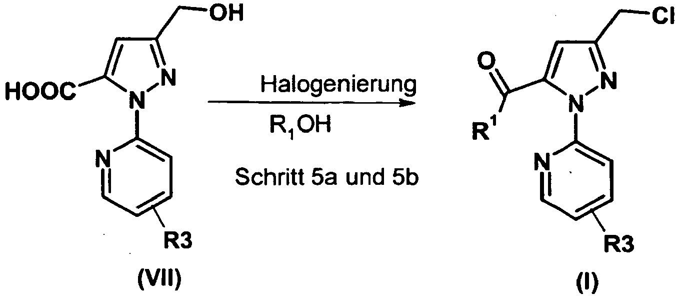

Zuerst werden die Verbindungen der Formel (VII) mit einem Halogenierungsmittel zu den entsprechenden Säurehalogeniden umgewandelt. Gleichzeitig findet auch der Austausch der Hydroxy-Gruppe durch Halogen statt. 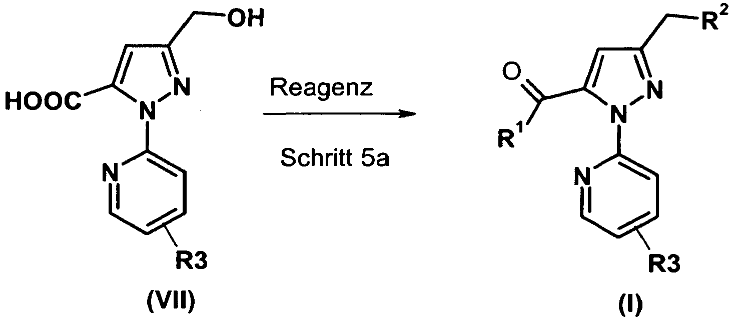 in welcher R¹ für Halogen und R² für Chlor, Brom, Jod, Fluor steht.

Die Verbindungen der Formel (I), in welcher R¹ für Halogen und R² für Chlor, Brom, Fluor, Jod steht, sind neu.

Zur Bildung der Säurehalogenide und zum Austausch von Hydroxy gegen Halogen sind folgende Reagenzien geeignet: SOCl₂, POCl₃, Oxalylchlorid, Phosgen, Diphosgen, POBr₃, PBr₃, SF₄, HCF₂CF₂N(Me)₂, PI₃. Bevorzugt sind SOCl₂, Oxalylchlorid , POCl₃, Phosgen.

Die Durchführung des erfindungsgemäßen Halogenierungssschritts (Schritt 5a) erfolgt vorzugsweise innerhalb eines Temperaturbereichs von -20°C bis +100°C, besonders bevorzugt bei Temperaturen von -10°C bis +70 °C.

Der erfindungsgemäße Verfahrensschritt wird im Allgemeinen unter Normaldruck durchgeführt. Alternativ ist es jedoch auch möglich, im Vakuum oder unter Überdrück (z.B. Umsetzung mit Phosgen) zu arbeiten.

Die Reaktionszeit ist nicht kritisch und kann, in Abhängigkeit von der Ansatzgröße und Temperatur, in einem Bereich zwischen wenigen Minuten und mehreren Stunden gewählt werden.

Bei der Durchführung des erfindungsgemäßen Verfahrensschritts setzt man 1 Mol der Säure der Formel (VII) mit 1.9 Mol bis 2.5 Mol, vorzugsweise 1.95 Mol bis 2.2 Mol, besonders bevorzugt mit der äquimolaren Menge (2 Eq) des Chlorierungssmittels um.

Geeignete Lösungsmittel sind beispielsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan, Nitrile, wie Acetonitril, Propionitril, n- oder iso-Butyronitril oder Benzonitril; Amide, wie N,N-Dimethyl-formamid, N,N-Dimethylacetamid, N-Methylformanilid, N-Methylpyrrolidon oder Hexamethylphosphorsäuretriamid. Besonders bevorzugt verwendet man Toluol, Xylol, Chlorbenzol, n-Hexan, Cyclohexan oder Methylcyclohexan, Methylenchlorid, Dichlorethan, ganz besonders bevorzugt Toluol, Xylol.

Im **Schritt 5b** reagieren die Säurehalogenide mit Alkohol unter Bildung von Estern der Formel (I).

Bevorzugt sind die Alkohole wie : Methanol, Ethanol, Propanol, i-Propanol, Cyclohexanol.

Die Durchführung des erfindungsgemäßen Verfahrensschritts erfolgt vorzugsweise innerhalb eines Temperaturbereichs von -20°C bis +100°C, besonders bevorzugt bei Temperaturen von -10°C bis +40 °C.

Die Reaktionszeit ist nicht kritisch und kann, in Abhängigkeit von der Ansatzgröße und Temperatur, in einem Bereich zwischen wenigen Minuten und mehreren Stunden gewählt werden.

Bei der Durchführung des erfindungsgemäßen Verfahrensschritts setzt man 1 Mol des Säurehalogenids der Formel (VII) mit 1 bis 3 Eq, bevorzugt 1 Eq. des Alkohols. Die Reaktion kann in Alkohol als Lösemitteln durchgeführt werden. Die Halogenierung und Umsetzung mit Alkohol werden in der Regel als Eintopfreaktion durchgeführt.

Die erfindungsgemäßen Verbindungen der Formel (I) werden sind wertvolle Zwischenprodukte in der Synthese von Anthranilsäureamiden (WO2007/112893, WO2007/144100).

### Herstellungsbeispiele:

### Beispiel 1

**5-(Benzyloxy)-1,1,1-trichloropent-3-yn-2-one, 5-(Benzyloxy)-1-bromo-1,1-dichloropent-3-yn-2-**one wurde aus Benzylpropargylether, Butyl-Li und CCl₃COOEt wie in THL 45(2004) 4935-4938 beschrieben hergestellt.

### Beispiel 2

### 3-[(Benzyloxy)methyl]-1-(3-chloropyridin-2-yl)-5-(trichloromethyl)-4,5-dihydro-1H-pyrazol-5-ol

5-(Benzyloxy)-1,1,1-trichloropent-3-yn-2-one (2.9 g., 0.01 mol) und 3-Chlor-2-hydrazinopyridine (1.43 g., 0.01 mol) wurden in 20 ml Methyltert-butylether vorgelegt (exotherm) und das Gemisch 1 Std. bei 30°C nachgerührt. Das Lösungsmittel wurde einrotiert und das resultierende Gemisch mittels LC/MS analysiert. Nur ein Isomer mit m/e 435 wurde identifiziert. Die Ausbeute betrug 94 %, die Reinheit 92 % (Flächenprozent).

Charakterisierung:
¹H NMR (CDCl3): 3.5 [(1H, d (19 Hz)]; 3.84 [(1H, d, 19 Hz)]; 4.32 (2H, s); 4.52 (m, 2H), 7.1 (1H, m); 7.3-7.4 (5H, m); 7.8 [(1H, d, 2 Hz.)]; 8.1 [(1H, d, 2 Hz)] ppm.
Schmelzpunkt (m.p.): 112-113°C.

### Beispiel 3

### 1-(3-Chloropyridin-2-yl)-3-[(tetrahydro-2H-pyran-2-yloxy)methyl]-5-(trichloromethyl)-4,5-dihydro-1H-pyrazol-5-ol, Mischung zweier Diastereomere

Anstelle von 5- (Benzyloxy)-1,1,1-trichloropent-3-yn-2-one (siehe Beispiel Nr. 2), wurde 1,1,1-trichloro 5-(tetrahydro-2H-pyran-2-yloxy)pent-3-yn-2-one eingesetzt. Die Herstellung erfolgte analog wie i Beispiel Nr. 2 beschrieben.

Charakterisierung des resultierenden Diastereomerengemisches:
¹H NMR (CDCl3): 1,46-1.58 (4H); 1,66-1,73 (1H, m); 1.75-1.8 (1H, m); 3,48 (1H, m); 3,81 (1H, m); 3,33 (1H,d); 3,81 (1H, d); 7,21 (1H, dd); 7,23 (1H, dd); 7,94 (1H, dd); 8,22 (1H, dd); 9,48 (1H, bs) ppm.

### Beispiel 4

### 2-{3-[(Benzyloxy)methyl]-5-(trichloromethyl)-1H-pyrazol-1-yl}-3-chloropyridine

4.35 g von 3-[(Benzyloxy)methyl]-1-(3-chloropyridin-2-yl)-5-(trichloromethyl)-4,5-dihydro-1H-pyrazol-5-ol wurden in 30 ml Methylisobutylether gelöst. Dann wurden 3 g Trifluoressigsäureanhydrid zugegeben (exothermische Reaktion). Das Gemisch wurde 2 Std. bei 25°C nachgerührt, wobei die Bildung des Niederschlags stattfand. Der Niederschlag wurde abfiltriert und gewaschen. Die Ausbeute betrug 95 %.

Charakterisierung:
¹H NMR (CDCl3): 4.60 (2H, s); 4.62 (m, 2H), 6.95 (1H, s); 7.2-7.4 (5H, m); 7.42 (1H,m); 7.95 [(1H,d 2 Hz.)]; 8.5 [(1H, d, 2 Hz)] ppm.
Schmelzpunkt (m.p.): 211-213°C.

### Beispiel 5

### 3-[(Benzyloxy)methyl]-1-(3-chloropyridin-2-yl)-1H-pyrazole-5-carboxylic acid

4.4 g von 2-{3-[(Benzyloxy)methyl]-5-(trichloromethyl)-1H-pyrazol-1-yl}-3-chloropyridine und 30 ml 20 % H₂SO₄ wurden 24 Std bei 100°C erhitzt.

Der Niederschlag wurde abfiltriert und mit Wasser gewaschen. Die Ausbeute betrug 92 %.

Charakterisierung:
¹H NMR (CDC13): 4.61 (2H, s); 4.63 (m, 2H), 6.97 (1H, s); 7.2-7.4 (5H, m); 7.42 (1H,m); 7.96 [(1H,d 2 Hz.)]; 8.5 [(1H, d, 2 Hz)] ppm.

### Beispiel 6

### 1-(3-chloropyridin-2-yl)-3-(hydroxymethyl)-1H-pyrazole-5-carboxylic acid hydrochlorid

3.43 g von 3-[(Benzyloxy)methyl]-1-(3-chloropyridin-2-yl)-1H-pyrazole-5-carboxylic acid und 20 ml HCl (37,5 %) wurden 2 Std bei 100°C erhitzt und dann das Reaktionsgemisch in Vakuum bei 10 mbar komplett eingeengt. Man erhielt 1-(3-chloropyridin-2-yl)-3-(hydroxymethyl)-1H-pyrazole-5-carboxylic acid als Hydrochlorid. Durch Neutralisation mit NaHCO₃ wurde die freie Säure als weißer Feststoff erhalten. Die Ausbeute betrug 94 %.

### Beispiel 7

### Methyl 3-(chlormethyl)-1-(3-chloropyridin-2-yl)-1H-pyrazole-5-carboxylate

1-(3-chloropyridin-2-yl)-3-(hydroxymethyl)-1H-pyrazole-5-carboxylic acid hydrochlorid (0.1 mol) wurde in 50 ml Toluol vorgelegt. SOCl₂ wurde portionsweise bei 60°C zugegeben. Man erhitzte das Gemisch 3 Std. bei 70°C, wobei der Niederschlag komplett in die Lösung ging. Methanol (30 ml) wurde langsam zu dem Gemisch zugetropft und die Lösung 1 Stunde bei Raumtemperatur nachgerührt. Anschließend wurde die Lösung im Vakuum eingeengt. Man erhielt 95% des Produktes mit einer Reinheit von 96 % (Flächenprozent).

Charakterisierung
¹H NMR (CDC13): 3.7 (3H,s); 4.7 (2H, s); 7.1 (1H, s); 7.5 (1H, m); 8.05 [(1H, m)]; 8.5 [(1H, m)] ppm.

## Patentansprüche

1. Verfahren zur Herstellung von Pyridyl-substituierten Pyrazol-Derivaten der allgemeinen Formel (1) 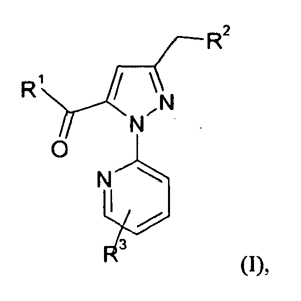 in welcher
R¹ für Alkoxy, Hydroxy, Aryloxy, Alkylaryloxy, Alkyl, Cycloalkyl, Halogen steht,
R² für Hydroxy, Alkoxy, Arylalkoxy, Alkylthio, für Chlor, Brom, Fluor, Jod, O-(C=O)Alkyl, O-(C=O)O-Alkyl, OSO₂Alkyl, OSO₂Ph, OSO₂-Halogenalkyl, OSO₂-Aryl steht,
**dadurch gekennzeichnet, dass** man
(A) Acetylenketone der Formel (II) 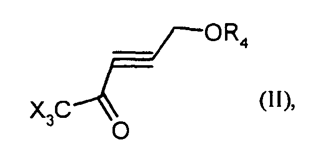 in welcher
R⁴ für eine Schutzgruppe steht, ausgewählt aus (C₁-C₆)-Alkyl, Aryl, Benzyl, Tetrahydropyran, (C=O)-Alkyl, (C=O)-OAlkyl, Si(Alkyl)₃
und X für Halogen steht,
mit Hydrazinopyridinen der Formel (III) 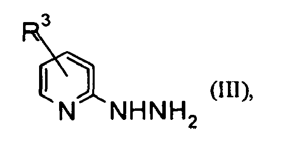 in welcher
R³ für Halogen, CN, NO₂, Alkyl, Cycloalkyl, Halogenalkyl, Halogencycloalkyl, Alkoxy, Halogenalkoxy, Alkylamino, Dialkylamino, Cycloalkylamino, steht,
umsetzt zu 1-Pyridyl-substituierten dihydro-1H Pyrazolen der Formel (IV), 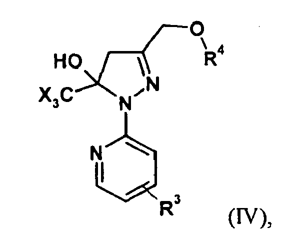 in welcher X, R³, R⁴ die oben angegebenen Bedeutungen haben,
(B) diese gegebenenfalls ohne vorherige Isolierung unter Wasserabspaltung weiter umsetzt zu 1-Pyridyl- substituierten Trihalogenmethylpyrazolen der Formel (V) 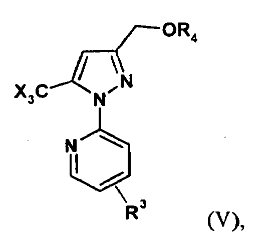 in welcher X, R³, R⁴ die oben angegebenen Bedeutungen haben,
(C) diese Verbindungen der allgemeinen Formel (V)
unter Zugabe von beispielsweise H₂SO₄ umsetzt zu Pyrazolcarbonsäuren der Formel (VI), 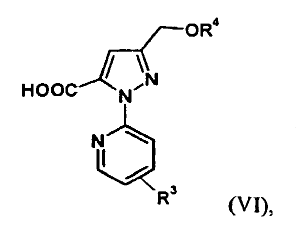 in welcher R³, R⁴ die oben angegebenen Bedeutungen haben,
(D) diese nach Abspaltung der Schutzgruppe R⁴ zu Hydroxymethylpyrazolsäuren der Formel (VII) umsetzt, 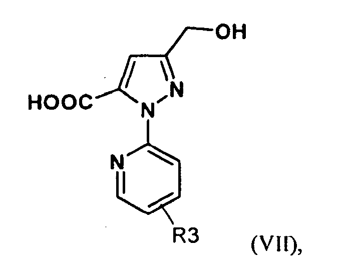 in welcher R³ die oben angegebenen Bedeutungen hat, und
(E) diese zu Verbindungen der Formel (I) umsetzt.

2. Verfahren zur Herstellung von Verbindungen der Formel (I) gemäß Anspruch 1, **dadurch gekennzeichnet, dass**
R¹ für (C₁-C₆)-Alkoxy, Halogen steht,
R² für (C₁-C₆)-Alkoxy, Aryl(C₁-C₆)-alkoxy, Fluor, Chlor, Brom, Jod steht,
R³ für Halogen, CN, NO₂, (C₁-C₆)-Alkyl, Halogen(C₁-C₆)-alkyl, (C₁-C₆)Alkoxy, Halogen(C₁-C₆)alkoxy steht.

3. Verfahren zur Herstellung von Verbindungen der Formel (I), **dadurch gekennzeichnet, dass** die Herstellung der Verbindung der Formel (V) die Schritte (A) und (B) gemäß Anspruch 1 umfasst.

4. Verfahren zur Herstellung von Verbindungen der Formel (I) gemäß Anspruch 3, **dadurch gekennzeichnet, dass** die Verbindungen der Formel (V), 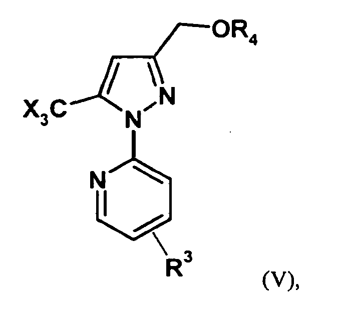 in welcher
X für Halogen,
R⁴ für eine Schutzgruppe steht, ausgewählt aus (C₁-C₆)-Alkyl, Aryl, Benzyl, Tetrahydropyran, (C=O)-Alkyl, (C=O)-OAlkyl, Si(Alkyl)₃
R³ für Halogen, CN, NO₂, Alkyl, Cycloalkyl, Halogenalkyl, Halogencycloalkyl, Alkoxy, Halogenalkoxy, Alkylamino, Dialkylamino, Cycloalkylamino steht,
durch Abspaltung der Schutzgruppe umgesetzt werden zu Verbindungen der Formel (VIII), 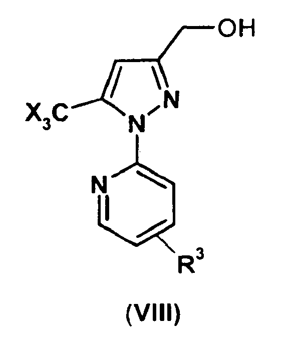 in welcher
X, R³ die oben angegebenen Bedeutungen haben,
diese unter Hydrolyse umsetzt zu Verbindungen der allgemeinen Formel (VII), 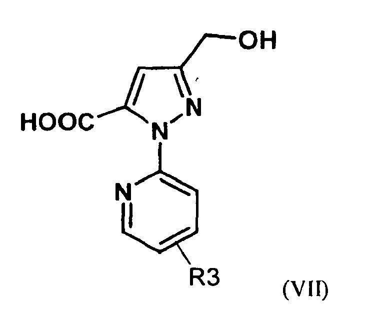 in welcher
R³ die oben angegebenen Bedeutungen hat,
und diese unter Zugabe eines Halogenierungsmittels und anschließender Alkoholzugabe umsetzt zu Verbindungen der Formel (I).

5. Verfahren zur Herstellung von Verbindungen der Formel (I) gemäß Anspruch 3,
**dadurch gekennzeichnet, dass** die Verbindungen der Formel (V),
in welcher
X für Halogen,
R⁴ für eine Schutzgruppe steht, ausgewählt aus (C₁-C₆)-Alkyl, Aryl, Benzyl, Tetrahydropyran, (C=O) Alkyl, (C=O)-OAlkyl, Si(Alkyl)₃
R³ für Halogen, CN, NO₂, Alkyl, Cycloalkyl, Halogenalkyl, Halogencycloalkyl, Alkoxy, Halogenalkoxy, Alkylamino, Dialkylamino, Cycloalkylamino steht,
unter Zugabe von Alkohol direkt in die erfindungsgemäßen Verbindungen der Formel (I) umgesetzt werden.

6. Verbindungen der Formel (IV) 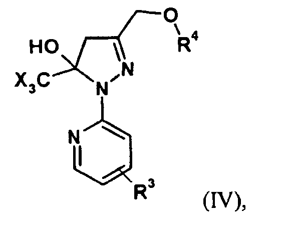 **dadurch gekennzeichnet, dass**
X für Halogen steht,
R³ für Chlor steht,
R⁴ für Benzyl steht.

7. Verbindungen der Formel (V) 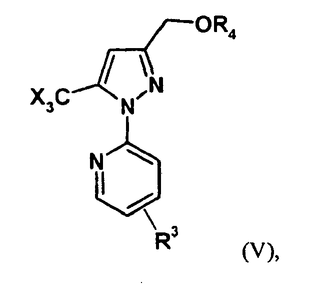 **dadurch gekennzeichnet, dass**
X für Halogen steht,
R³ für Chlor steht,
R⁴ für Benzyl steht.

8. Verbindungen der Formel (V) gemäß Anspruch 7, **dadurch gekennzeichnet, dass**
X für Chlor steht.

9. Verbindungen der Formel (VII), 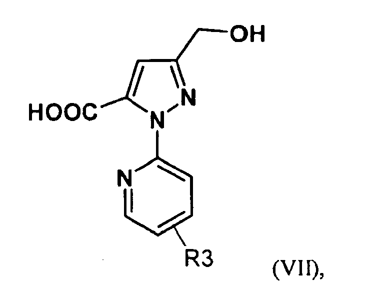 **dadurch gekennzeichnet, dass**
R³ für Halogen, CN, NO₂, Alkyl, Cycloalkyl, Halogenalkyl, Halogencycloalkyl, Alkoxy, Halogenalkoxy, Alkylamino, Dialkylamino, Cycloalkylamino steht.

## Claims

1. Process for preparing pyridyl-substituted pyrazole derivatives of the general formula (I) 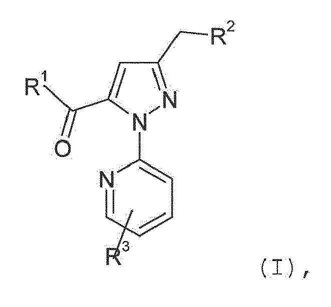 in which
R¹ is alkoxy, hydroxyl, aryloxy, alkylaryloxy, alkyl, cycloalkyl, halogen,
R² is hydroxyl, alkoxy, arylalkoxy, alkylthio, chlorine, bromine, fluorine, iodine, O-(C=O)alkyl, O-(C=O)O-alkyl, OSO₂alkyl, OSO₂Ph, OSO₂-haloalkyl, OSO₂-aryl,
**characterized in that**
(A) acetyleneketones of the formula (II) 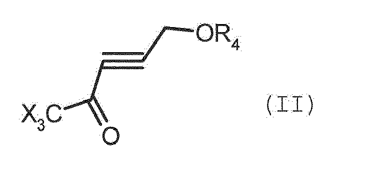 in which
R⁴ is a protecting group selected from (C₁-C₆)-alkyl, aryl, benzyl, tetrahydropyran, (C=O)-alkyl, (C=O)-Oalkyl, Si(alkyl)₃.
and X is halogen
are reacted with hydrazinopyridines of the formula (III) 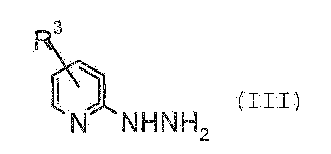 in which
R³ is halogen, CN, NO₂, alkyl, cycloalkyl, haloalkyl, halocycloalkyl, alkoxy, haloalkoxy, alkylamino, dialkylamino, cycloalkylamino,
to give 1-pyridyl-substituted dihydro-1H-pyrazoles of the formula (IV) 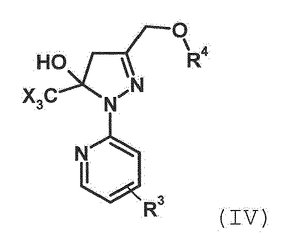 in which X, R³, R⁴ are each as defined above,
(B) the latter are optionally converted further, without preceding isolation, with elimination of water, to 1-pyridyl-substituted trihalomethylpyrazoles of the formula (V) 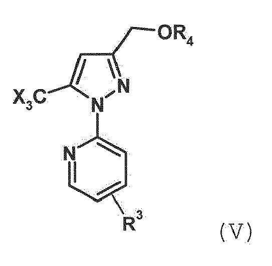 in which X, R³, R⁴ are each as defined above,
(C) these compounds of the general formula (V)
are converted with addition of H₂SO₄, for example, to pyrazolecarboxylic acids of the formula (VI) 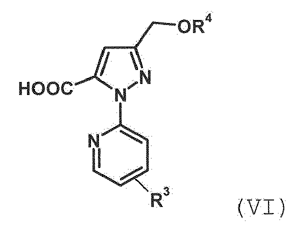 in which R³, R⁴ are each as defined above,
(D) the latter are converted, after detaching the protecting group R⁴, to hydroxymethylpyrazole acids of the formula (VII) 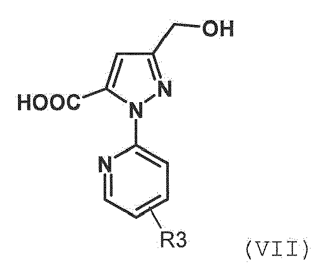 in which R³ is as defined above, and
(E) the latter are converted to compounds of the formula (I).

2. Process for preparing compounds of the formula (I) according to Claim 1, **characterized in that**
R¹ is (C₁-C₆)-alkoxy, halogen,
R² is (C₁-C₆)-alkoxy, aryl(C₁-C₆)-alkoxy, fluorine, chlorine, bromine, iodine,
R³ is halogen, CN, NO₂, (C₁-C₆)-alkyl, halo(C₁-C₆)-alkyl, (C₁-C₆)-alkoxy, halo (C₁-C₆)alkoxy.

3. Process for preparing compounds of the formula (I), **characterized in that** the preparation of the compound of the formula (V) comprises steps (A) and (B) according to Claim 1.

4. Process for preparing compounds of the formula (I) according to Claim 3, **characterized in that** the compounds of the formula (V) 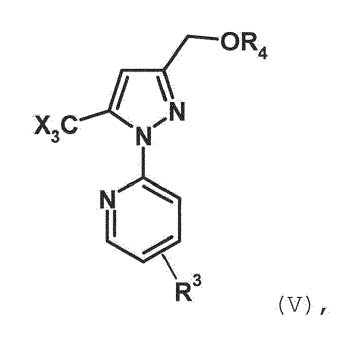 in which
X is halogen,
R⁴ is a protecting group selected from (C₁-C₆)-alkyl, aryl, benzyl, tetrahydropyran, (C=O)-alkyl, (C=O)-Oalkyl, Si(alkyl)₃,
R³ is halogen, CN, NO₂, alkyl, cycloalkyl, haloalkyl, halocycloalkyl, alkoxy, haloalkoxy, alkylamino, dialkylamino, cycloalkylamino,
are converted by detaching the protecting group to compounds of the formula (VIII) 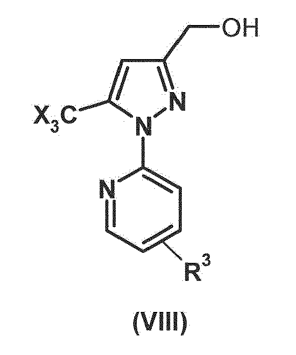 in which
X, R³ are each as defined above,
the latter are converted by hydrolysis to compounds of the general formula (VII) 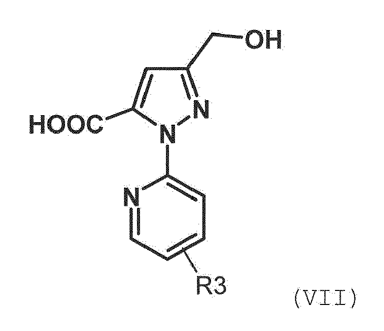 in which
R³ is as defined above,
and the latter are converted with addition of a halogenating agent and subsequent alcohol addition to compounds of the formula (I).

5. Process for preparing compounds of the formula (I) according to Claim 3,
**characterized in that** the compounds of the formula (V)
in which
X is halogen,
R⁴ is a protecting group selected from (C₁-C₆)-alkyl, aryl, benzyl, tetrahydropyran, (C=O)-alkyl, (C=O)-Oalkyl, Si(alkyl)₃,
R³ is halogen, CN, NO₂, alkyl, cycloalkyl, haloalkyl, halocycloalkyl, alkoxy, haloalkoxy, alkylamino, dialkylamino, cycloalkylamino,
are converted with addition of alcohol directly to the inventive compounds of the formula (I).

6. Compounds of the formula (IV) 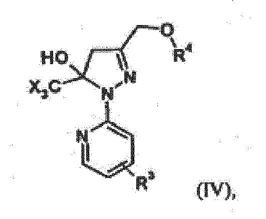 **characterized in that**
X is halogen,
R³ is chlorine,
R⁴ is benzyl.

7. Compounds of the formula (V) 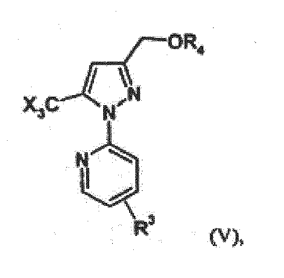 **characterized in that**
X is halogen,
R³ is chlorine,
R⁴ is benzyl.

8. Compounds of the formula (V) according to Claim 7, **characterized in that**
X is chlorine.

9. Compounds of the formula (VII) 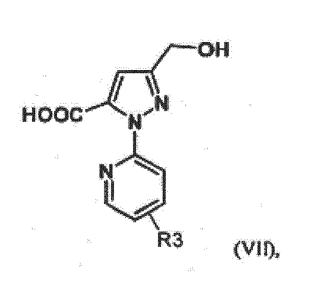 **characterized in that**
R³ is halogen, CN, NO₂, alkyl, cycloalkyl, haloalkyl, halocycloalkyl, alkoxy, haloalkoxy, alkylamino, dialkylamino, cycloalkylamino.

## Revendications

1. Procédé de fabrication de dérivés de pyrazole à substitution pyridyle de formule générale (I) 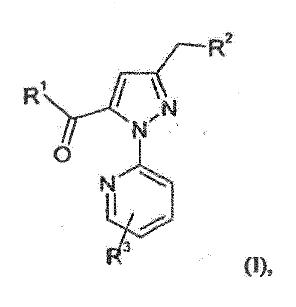 dans laquelle
R¹ représente alcoxy, hydroxy, aryloxy, alkylaryloxy, alkyle, cycloalkyle, halogène,
R² représente hydroxy, alcoxy, arylalcoxy, alkylthio, chlore, brome, fluor, iode, O-(C=O)alkyle, O-(C=O)O-alkyle, OSO₂alkyle, OSO₂Ph, OSO₂-halogénoalkyle, OSO₂-aryle,
**caractérisé en ce que**
(A) des acétylène-cétones de formule (II) 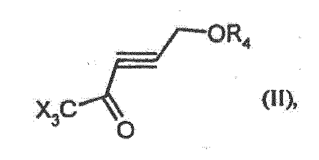 dans laquelle
R⁴ représente un groupe protecteur choisi parmi alkyle en (C₁-C₆), aryle, benzyle, tétrahydropyrane, (C=O)-alkyle, (C=O)-Oalkyle, Si(alkyle)₃ et X représente halogène,
sont mises en réaction avec des hydrazinopyridines de formule (III) 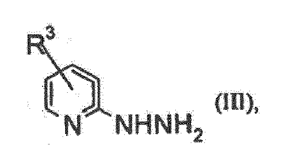 dans laquelle
R³ représente halogène, CN, NO₂, alkyle, cycloalkyle, halogénoalkyle, halogénocycloalkyle, alcoxy, halogénoalcoxy, alkylamino, dialkylamino, cycloalkylamino,
pour former des dihydro-1H-pyrazoles à substitution 1-pyridyle de formule (IV) 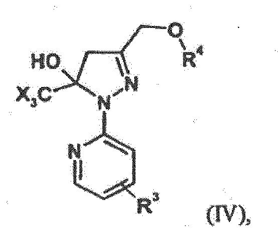 dans laquelle X, R³, R⁴ ont les significations indiquées précédemment,
(B) ceux-ci sont davantage transformés, éventuellement sans isolément préalable, par clivage d'eau, en trihalogénométhylpyrazoles à substitution 1-pyridyle de formule (V) 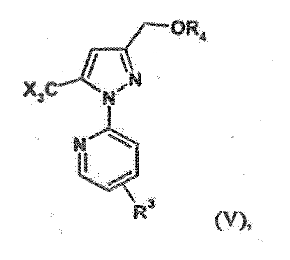 dans laquelle X, R³, R⁴ ont les significations indiquées précédemment,
(C) ces composés de formule générale (V) sont transformés par ajout par exemple d'H₂SO₄ en acides pyrazole-carboxyliques de formule (VI) 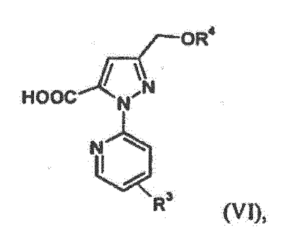 dans laquelle R³, R⁴ ont les significations indiquées précédemment,
(D) ceux-ci sont transformés, après clivage du groupe protecteur R⁴, en acides hydroxyméthylpyrazoliques de formule (VII) 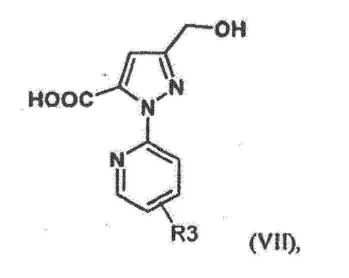 dans laquelle R³ a les significations indiquées précédemment, et
(E) ceux-ci sont transformés en composés de formule (I).

2. Procédé de fabrication de composés de formule (I) selon la revendication 1, **caractérisé en ce que**
R¹ représente alcoxy en (C₁-C₆), halogène,
R² représente alcoxy en (C₁-C₆), aryl-alcoxy en (C₁-C₆), fluor, chlore, brome, iode,
R³ représente halogène, CN, NO₂, alkyle en (C₁-C₆), halogéno-alkyle en (C₁-C₆), alcoxy en (C₁-C₆), halogéno-alcoxy en (C₁-C₆).

3. Procédé de fabrication de composés de formule (I), **caractérisé en ce que** la fabrication du composé de formule (V) comprend les étapes (A) et (B) selon la revendication 1.

4. Procédé de fabrication de composés de formule (I) selon la revendication 3, **caractérisé en ce que** les composés de formule (V) 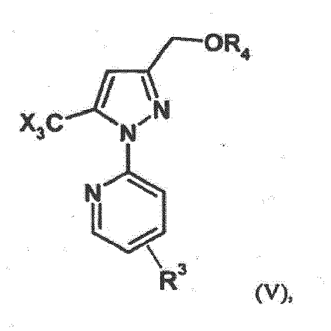 dans laquelle
X représente halogène,
R⁴ représente un groupe protecteur choisi parmi alkyle en (C₁-C₆), aryle, benzyle, tétrahydropyrane, (C=O)-alkyle, (C=O)-Oalkyle, Si(alkyle)₃,
R³ représente halogène, CN, NO₂, alkyle, cycloalkyle, halogénoalkyle, halogénocycloalkyle, alcoxy, halogénoalcoxy, alkylamino, dialkylamino, cycloalkylamino,
sont transformés par clivage du groupe protecteur en composés de formule (VIII) 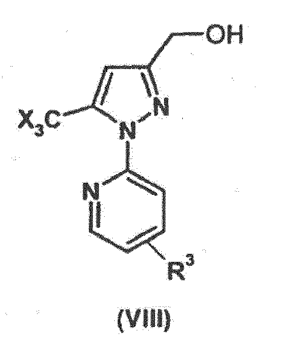 dans laquelle
X, R³ ont les significations indiquées précédemment, ceux-ci sont transformés par hydrolyse en composés de formule générale (VII) 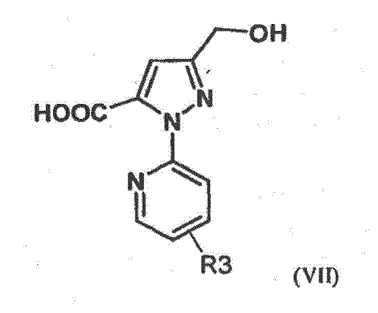 dans laquelle
R³ a les significations indiquées précédemment,
et ceux-ci sont transformés par ajout d'un agent d'halogénation, puis ajout d'un alcool en composés de formule (I).

5. Procédé de fabrication de composés de formule (I) selon la revendication 3, **caractérisé en ce que** les composés de formule (V)
dans laquelle
X représente halogène,
R⁴ représente un groupe protecteur choisi parmi alkyle en (C₁-C₆), aryle, benzyle, tétrahydropyrane, (C=O)-alkyle, (C=O)-Oalkyle, Si(alkyle)₃,
R³ représente halogène, CN, NO₂, alkyle, cycloalkyle, halogénoalkyle, halogénocycloalkyle, alcoxy, halogénoalcoxy, alkylamino, dialkylamino, cycloalkylamino,
sont transformés par ajout d'un alcool directement en les composés de formule (I) selon l'invention.

6. Composés de formule (IV) 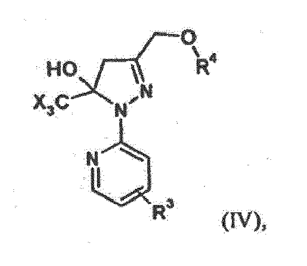 **caractérisés en ce que**
X représente halogène,
R³ représente chlore,
R⁴ représente benzyle.

7. Composés de formule (V) 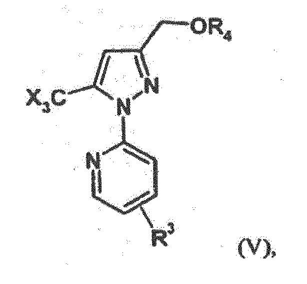 **caractérisés en ce que**
X représente halogène,
R³ représente chlore,
R⁴ représente benzyle.

8. Composés de formule (V) selon la revendication 7, **caractérisés en ce que**
X représente chlore.

9. Composés de formule (VII) 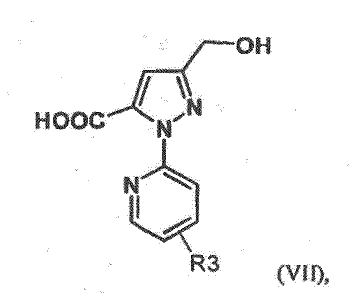 **caractérisés en ce que**
R³ représente halogène, CN, NO₂, alkyle, cycloalkyle, halogénoalkyle, halogénocycloalkyle, alcoxy, halogénoalcoxy, alkylamino, dialkylamino, cycloalkylamino.
